# EUROPEAN PATENT SPECIFICATION

(11) **EP 2 524 713 B1**
(45) Date of publication and mention of the grant of the patent: **12.10.2016**
(21) Application number: 11305600.6
(22) Date of filing: 18.05.2011
(51) Int. Cl.: A61M 16/04, A61B 1/267

(54) **System for intubation**
System zur Intubation
Système d'intubation correspondant

(43) Date of publication of application: 21.11.2012
(73) Proprietor: Centre Hospitalier Universitaire de Bordeaux, 33404 Talence (FR)
(72) Inventor: Rozé, Hadrien, 33000, BORDEAUX (FR)
(74) Representative: Novagraaf Technologies

(56) References cited:
- EP-A2- 1 977 678
- DE-A1-102007 049 191
- DE-U1-202006 017 519
- GB-A- 2 397 229
- US-A- 4 846 153
- US-A- 5 363 838
- US-A- 6 086 529
- US-A1- 2003 078 476
- US-A1- 2004 035 429
- US-A1- 2005 177 024
- US-A1- 2009 192 350
- US-B1- 6 609 521
- US-B1- 7 473 219

## Description

The present invention relates to the ventilation of at least one lung of an individual, through endotracheal intubation.

It is used as a temporary artificial airway in patient requiring mechanical ventilation, and intended for oral intubation procedures.

More specifically, the present invention relates to the positioning of a disposable sterile endotracheal tube into an individual's body.

Endotracheal intubation is a common technique that is used when an individual (hereafter referred to as the patient) must be ventilated such as after receiving a general anesthetic. The technique consists of placing an endotracheal tube into the patient's trachea to ventilate his/her lungs.

During intubation, the endotracheal tube must be inserted properly, in order to ventilate one or both of the patient's lungs.

Since the endotracheal tube must be inserted into the trachea, in a blind way, past the epiglottis and the vocal cords, several optical devices using fiber optics have been developed to overcome this issue.

A wrong positioning of the endotracheal tube can be life threatening. In particular, it can be required to insert a fiberscope into the endotracheal tube to control the positioning of the tube.

However, the use of an endobronchial fiberscope faces several issues. First, the cost of such apparatus is very high (today the price can be up to several tens of thousand Euros). Then, a fiberscope contains handles that are cumbersome when in use in a conjunction with endotracheal tubes since requiring then four hands. In addition, a fiberscope is somehow heavy. With regard to hygiene, the whole fiberscope needs to be sterilized, which requires a specific, costly, cumbersome apparatus, the sterilizing process being time consuming. In addition, it is worth noticing that a fiberscope contains a suction lumen that can also be a hygiene issue. For these reasons, the use of a fiberscope is dedicated to structures like hospitals and can not be used by emergency mobile units "in the field". DE 202006017519 describes an endo-tracheal tube having a fibre bandle only when not is ventilated.

In that context, the present invention aims to provide a solution allowing ventilating a patient while controlling the positioning of an endotracheal tube with optical means.

To this end, a first object of the present invention relates to a system for intubation comprising:
- a disposable sterile endotracheal tube having a proximal end and a distal end designed to be inserted into a patient's body.
According to the invention, the system is mainly characterized in that it further comprises:
- a disposable removable sterile optical fiber bundle having a proximal end and a distal end, and comprising a first optical fiber, and a second optical fiber, said optical fiber bundle being designed to be inserted into said endotracheal tube, so that said distal end of said bundle is capable of illuminating a region of said distal end of said endotracheal tube, and of collecting an image of said illuminated region, and
- a module comprising a screen and a light source, said module being designed to be connected to the proximal end of said optical fiber bundle to provide light from said light source to the distal end of said optical fiber bundle via said first optical fiber, and to display on the screen said image collected from the distal end of said optical fiber bundle via said second optical fiber.
characterized in that:
the external diameter of said optical fiber bundle (20) is at least two times smaller than the diameter of the airway lumen (32) of said endotracheal tube (30), so that ventilation of the patient can occur while said optical fiber bundle (20) is inserted into said tracheal tube (30).

In one embodiment, said proximal end of said endotracheal tube further comprises an openable and closable seal to open/close an aperture through which said optical fiber bundle can be inserted into said endotracheal tube, said optical fiber bundle being secured to said endotracheal tube via said seal when inserted into said endotracheal tube.

In one embodiment, said module further comprises (rechargeable) batteries to provide energy to the screen and to the light source.

In one embodiment, the system according to the invention further comprises a laryngoscope, designed to be introduced into said patient's mouth and upper airway to aid passing said endotracheal tube into the trachea of said patient.

In one embodiment, the external diameter of said optical fiber bundle is at least two times smaller than the internal diameter, i.e. the airway lumen, of said endotracheal tube.

In one embodiment, said endotracheal tube is a one-lung ventilation endotracheal tube.

In one embodiment, said endotracheal tube further comprises a carina hook.

Another object relates to a method for positioning a disposable sterile endotracheal tube according to the invention into a patient's body, said method comprising:
- inserting said endotracheal tube into said patient's body, and
- ventilating said patient via said endotracheal tube,
The method according to the invention is essentially characterized in that it further comprises:
- inserting into said endotracheal tube a disposable removable sterile optical fiber bundle having a proximal end and a distal end, and comprising a first optical fiber, and a second optical fiber,
- connecting the proximal end of said optical fiber bundle to a module comprising a screen and a light source,
- providing light from said light source to the distal end of said optical fiber bundle via said first optical fiber, and illuminating a region of the distal end of said endotracheal tube with the distal end of said optical fiber bundle,
- collecting an image of said illuminated region via said second optical fiber of said optical fiber bundle,
- displaying on the screen said collected image.

In one embodiment, the ventilation of the patient is selective ventilation, said endotracheal tube being a one-lung ventilation endotracheal tube.

In one embodiment, said endotracheal tube comprises a carina hook, said method comprising:
- positioning/repositioning of the carina hook on the carina of said patient.

In one embodiment, the method according to the invention further comprises:
- removing said disposable removable sterile optical fiber bundle from said endotracheal tube.

In one embodiment, the method according to the invention further comprises:
- inserting into said endotracheal tube another disposable removable sterile optical fiber bundle.

In one embodiment, the method according to the invention further comprises:
- introducing a laryngoscope into said patient's mouth and upper airway to aid passing said endotracheal tube into the trachea of said patient.

In one embodiment, said disposable removable sterile optical fiber bundle is inserted into said endotracheal tube via an openable and closable seal disposed on the proximal end of said endotracheal tube.

In one embodiment, the step of inserting a disposable removable sterile optical fiber bundle into said endotracheal tube is simultaneous with the step of inserting said endotracheal tube into said patient's body.

Thanks to the invention, a real-time verification of tube position is possible.

Other features and advantages of the present invention will appear throughout the following description, wherein examples shall be considered as merely illustrative and non restrictive, with reference to the accompanying drawings in which:

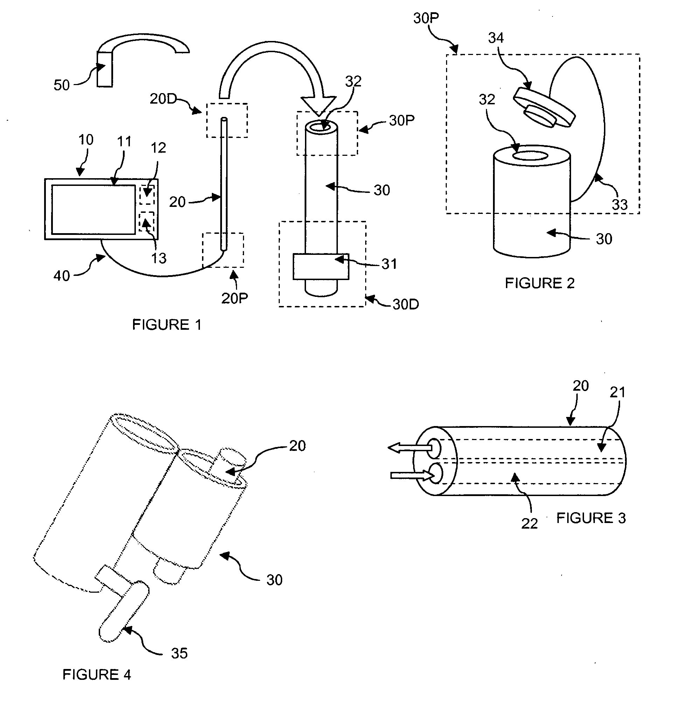

- Fig. 1 illustrates an embodiment of a system according to the invention,
- Fig. 2 illustrates an embodiment of a seal on the proximal end of an endotracheal tube in a system according to the invention,
- Fig. 3 illustrates an embodiment of a fiber bundle in a system according to the invention,
- Fig. 4 illustrates a part of a fiber bundle inserted into a double lumen endotracheal tube in a system according to the invention,
- Fig. 5 illustrates an embodiment of a method according to the invention.

A system for intubation comprises several elements. According to the proposed solution, each element can easily be found on the market place.

One element of the system is a disposable sterile endotracheal tube 30. Such tube 30 comprises a proximal end 30P and a distal end 30D, said distal end 30D being designed to be inserted into a patient's body.

In one embodiment, said proximal end 30P of said endotracheal tube 30 further comprises an openable and closable seal 34 to open/close an aperture through which said optical fiber bundle 20 can be inserted into the airway lumen 32 of said endotracheal tube, through a membrane (not illustrated). The seal 34 is preferably secured to the tube endotracheal 30 via a link 33. The seal 34 is used as an air sealing of the tube 30. Generally, the probe has an axis of elongation, said seal 34 being perpendicular to said axis of elongation so that the fiber bundle can be inserted parallel to said axis of elongation.

The optical fiber bundle 20 can then be secured to said endotracheal tube 30 via said seal 34 when inserted into said endotracheal tube 30.

Each disposable removable sterile endotracheal tube 30 can be placed into a respective dedicated flexible pouch or jacket of thin-wall plastic, so as to be easily transportable, including in an ambulance.

An endotracheal tube 30 can be a one-lung ventilation (OLV) tube, including a double lumen tube (DLT), right or left such as Carlens or Robertshaw tubes respectively, with or without carina hook 35. Such tubes enable selective ventilation of a patient.

Another element of the system is a disposable removable sterile optical fiber bundle 20 having a proximal end 20P and a distal end 20D. At least the distal end 20D of the optical fiber bundle 20 is designed to be inserted into the endotracheal tube 30, so that both distal ends 20D, 30D are disposed in a same region of a patient's body. The distal end 20D of said bundle is then capable of illuminating said region of said distal end 30D of said endotracheal tube, and of collecting an image of said illuminated region.

A classical fiber bundle can be sterilized for this purpose. Such sterilization step is very easy to implement, especially compared to the sterilization of a fiberscope.

Each disposable removable sterile optical fiber bundle 20 can be placed into a respective dedicated flexible pouch or jacket of thin-wall plastic, so as to be easily transportable, including in an ambulance.

The fiber bundle 20 comprises a first optical fiber 21, and a second optical fiber 22. In one embodiment, the fiber bundle 20 comprises only one first optical fiber 21, and only one second optical fiber 22, so as to decrease as much as possible the diameter of the fiber bundle 20. The first optical fiber 21 is used e.g. to provide light from a light source 12 to the distal end 20D. The second optical fiber 22 is used e.g. to collect light from to the distal end 20D so that an image can be displayed on a screen 11 of a module 10.

The fiber bundle 20 is advantageously flexible. Therefore when inserted into the airway lumen 32 of the endotracheal tube 30, it is guided by the internal shape of the tube 30. Thus the optical means 20 according to the invention do not require handles like in a fiberscope. The system is then very handy, light and not cumbersome.

The external diameter of said optical fiber bundle 20 can be as low as 4 mm. The internal diameter of a woman double lumen endotracheal tube can be of about 8 mm. In one embodiment, it is then at least two times smaller than the internal diameter of the airway lumen 32 of said endotracheal tube 30, so that ventilation of the patient can occur while said optical fiber bundle 20 is inserted into said endotracheal tube 30. It will be understood that the shape of an internal endotracheal tube can not be circular but rather shaped as a D capital letter. Then the ratio between so called internal diameters shall be understood as cross sectional surfaces ratio between said optical fiber bundle and said internal endotracheal tube.

An optical fiber bundle 20 can be found on the marketplace.

Another element of the system is a module 10 comprising a visualization screen 11 and a light source 12 (disposed within the module 10), e.g. a LED light source.

The module 10 is designed to be connected to the proximal end 20P of said optical fiber bundle 20 to provide light from said light source 12 to the distal end 20D of said optical fiber bundle via said first optical fiber 21, and to display on the screen 11 an image collected from the distal end 20D of said optical fiber bundle via said second optical fiber 22. Not shown, the module also comprises an illumination channel in communication with the light source 12, to which the first optical fiber 21 can be connected to; and an image channel in communication with the screen, to which the second optical fiber 22 can be connected to.

A light sensor, usually CCD or CMOS, is also not illustrated and disposed preferably into the module 10. Such light sensor can also be disposed into the fiber bundle 20.

The module 10 preferably further comprises batteries 13 to provide energy to the screen 11 and to the light source 12. Preferably, batteries 13 are rechargeable ones.

The module 10 is e.g. a LCD screen portable device that can be found on the marketplace.

The system can comprise a laryngoscope 50, designed to be introduced into said patient's mouth and upper airway to aid passing said endotracheal tube 30 into the trachea of the patient.

In operation, the endotracheal tube 30 is inserted 100 into a patient's body.

To this end, a laryngoscope 50 is preferably introduced 105 into said patient's mouth and upper airway to aid passing said endotracheal tube 30 into the trachea of said patient.

Said patient is then ventilated 200 via said endotracheal tube 30.

To this end, the proximal end 30P of the endotracheal tube 30 is connected, via an adaptor, to a machine such as a ventilator, which provides air to the patient's lungs.

Once the endotracheal tube 30 is inserted into a patient's body, a disposable removable sterile optical fiber bundle 20 is inserted 110 into the airway lumen 32 of said endotracheal tube 30. Preferably, the distal end 20D of the optical fiber bundle 20 meet the distal end 30D of the endotracheal tube 30.

The proximal end 20P of said optical fiber bundle 20 is connected 120 to a module 10 comprising a screen 11 and a light source 12. To this end, the proximal end 20P comprises e.g. a universal adaptor (non illustrated) like a jack or the like, to be plugged into a corresponding plug of the module 10. Preferably, the whole optical fiber bundle 20 including its adaptor is sterilized beforehand.

The light is provided 130 from a light source 12 the module 10 to the distal end 20D of said optical fiber bundle 20 via a first optical fiber 21, so as to illuminate a region of the distal end 30D of said endotracheal tube 30.

An image of said illuminated region can then be collected via a second optical fiber 22 of said optical fiber bundle 20, and displayed 140 on the screen 11 of said module 10, while the patient keep being ventilated.

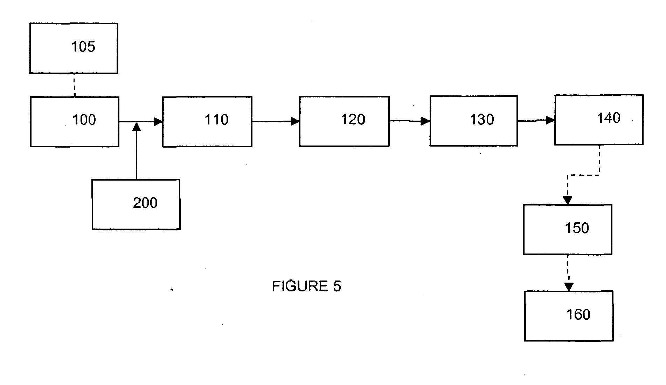

The sterile optical fiber bundle 20 can be removed 150 from said endotracheal tube 30, such option being illustrated with dashed lines on Fig. 5.

I needed, the same or another disposable removable sterile optical fiber bundle 20 can be inserted 160 into said endotracheal tube 30. For example, a disposable removable sterile optical fiber bundle 20 can be inserted every time it is needed to check to position of the endotracheal tube 30.

When the endotracheal tube 30 comprises a carina hook 35, the method can comprise the positioning/repositioning 170 of the carina hook 35 on the carina of said patient. For example, a disposable removable sterile optical fiber bundle 20 can be inserted every time it is needed to check to position of the carina hook.

The optical fiber bundle 20 can be inserted into the airway lumen 32 of the endotracheal tube 30 simultaneously with the insertion of the endotracheal tube 30 into said patient. This enables to get a real time image of the position of the distal end 30D of the tube 30, e.g. to help passing the glottis of the patient.

The method according to the invention is preferably related to a selective ventilation of a patient.

Thanks to the disposable sterile feature of the optical fiber bundle 20, said fiber bundle 20 can be inserted into the airway lumen 32 of the endotracheal tube 30 only for a short period of time, thus no water vapour can obstruct the distal end 20D of the fiber bundle 20, and a better average ventilation of the patient is obtained.

Thanks to the external diameter of the optical fiber bundle 20 vs. the diameter of the airway lumen 32 of the endotracheal tube 30, the patient can keep being ventilated while an image is displayed in real time.

According to the fact that the image displayed on the screen 11 of the module 10 does not need to be of high quality, low cost optical fiber bundle 20 can be used. Then the cost of use of the system according to the invention is much lower than the use of a fiberscope.

## Claims

1. System for intubation comprising:
- a disposable sterile endotracheal tube (30) having a proximal end (30P) and a distal end (30D) designed to be inserted into a patient's body,
- a disposable flexible removable sterile optical fiber bundle (20) having a proximal end (20P) and a distal end (20D), and comprising a first optical fiber (21), and a second optical fiber (22), said optical fiber bundle (20) being designed to be inserted into the airway lumen (32) of said endotracheal tube (30), so that said distal end (20D) of said bundle (20) is capable of illuminating a region of said distal end (30D) of said endotracheal tube (30), and of collecting an image of said illuminated region, and
- a module (10) comprising a screen (11) and a light source (12), said module (10) being designed to be connected to the proximal end (20P) of said optical fiber bundle (20) to provide light from said light source (12) to the distal end (20D) of said optical fiber bundle (20) via said first optical fiber (21), and to display on the screen (11) said image collected from the distal end (20D) of said optical fiber bundle (20) via said second optical fiber (22),
**characterized in that**:
the external diameter of said optical fiber bundle (20) is at least two times smaller than the diameter of the airway lumen (32) of said endotracheal tube (30), so that ventilation of the patient can occur while said optical fiber bundle (20) is inserted into said endotracheal tube (30).

2. System according to claim 1, wherein said proximal end (30P) of said endotracheal tube (30) further comprises an openable and closable seal (34), said optical fiber bundle (20) being secured to said endotracheal tube (30) via said seal (34) when inserted into said endotracheal tube (30).

3. System according to any preceding claims, wherein said module (10) further comprises batteries (13) to provide energy to the screen (11) and to the light source (12).

4. System according to any preceding claims, further comprising a laryngoscope (50), designed to be introduced into said patient's mouth and upper airway to aid passing said endotracheal tube (30) into the trachea of said patient.

5. System according to any preceding claims, wherein the external diameter of said optical fiber bundle(20) is as low as 4 mm.

6. System according to any preceding claims, wherein said endotracheal tube (30) is a one-lung ventilation endotracheal tube.

7. System according to any claim 6, wherein said endotracheal tube (30) further comprises a carina hook (35).

## Patentansprüche

1. System zur Intubation, umfassend:
- einen sterilen Einweg-Endotrachealtubus (30) mit einem proximalen Ende (30P) und einem distalen Ende (30D), das ausgebildet ist, um in den Körper eines Patienten eingeführt zu werden,
- ein flexibles entfernbares steriles optisches Einweg-Faserbündel (20) mit einem proximalen Ende (20P) und einem distalen Ende (20D) und umfassend eine erste optische Faser (21) und eine zweite optische Faser (22), wobei das optische Faserbündel (20) ausgebildet ist, um in das Atemweglumen (32) des Endotrachealtubus (30) eingeführt zu werden, so dass das distale Ende (20D) des Bündels (20) dazu in der Lage ist, einen Bereich des distalen Endes (30D) des Endotrachealtubus (30) zu beleuchten und ein Bild des beleuchteten Bereichs aufzunehmen, und
- ein Modul (10), umfassend einen Bildschirm (11) und eine Lichtquelle (12), wobei das Modul (10) ausgebildet ist, um mit dem proximalen Ende (20P) des optischen Faserbündels (20) verbunden zu sein, um Licht von der Lichtquelle (12) dem distalen Ende (20D) des optischen Faserbündels (20) über die erste optische Faser (21) bereitzustellen und auf dem Bildschirm (11) das Bild anzuzeigen, das vom distalen Ende (20D) des optischen Faserbündels (20) über die zweite optische Faser (22) aufgenommen wurde,
**dadurch gekennzeichnet, dass**:
der Außendurchmesser des optischen Faserbündels (20) mindestens zweimal kleiner als der Durchmesser des Atemweglumens (32) des Endotrachealtubus (30) ist, so dass die Ventilation des Patienten erfolgen kann, während das optische Faserbündel (20) in den Endotrachealtubus (30) eingeführt wird.

2. System nach Anspruch 1, wobei das proximale Ende (30P) des Endotrachealtubus (30) weiter ein zu öffnendes und zu verschließendes Siegel (34) umfasst, wobei das optische Faserbündel (20) an den Endotrachealtubus (30) über das Siegel (34) befestigt wird, wenn es in den Endotrachealtubus (30) eingeführt wird.

3. System nach einem der vorhergehenden Ansprüche, wobei das Modul (10) weiter Batterien (13) umfasst, um dem Bildschirm (11) und der Lichtquelle (12) Energie bereitzustellen.

4. System nach einem der vorhergehenden Ansprüche, weiter umfassend ein Laryngoskop (50), das ausgebildet ist, um in den Mund und den oberen Atemweg des Patienten eingeführt zu werden, um dabei zu helfen, den Endotrachealtubus (30) in die Luftröhre des Patienten einzuführen.

5. System nach einem der vorhergehenden Ansprüche, wobei der Außendurchmesser des optischen Faserbündels (20) nur 4 mm groß ist.

6. System nach einem der vorhergehenden Ansprüche, wobei der Endotrachealtubus (30) ein Ein-Lungen-Ventilations-Endotrachealtubus ist.

7. System nach Anspruch 6, wobei der Endotrachealtubus (30) weiter einen Karinahaken (35) umfasst.

## Revendications

1. Système d'intubation comprenant :
- un tube endotrachéal stérile jetable (30) ayant une extrémité proximale (30P) et une extrémité distale (30D), conçu pour être inséré dans le corps d'un patient,
- un faisceau (20) de fibres optiques jetable, flexible, stérile, pouvant être retiré ayant une extrémité proximale (20P) et une extrémité distale (20D), et comprenant une première fibre optique (21) et une deuxième fibre optique (22), ledit faisceau (20) de fibres optiques étant conçu pour être inséré dans le lumen d'une voie respiratoire (32) dudit tube endotrachéal (30), de sorte que ladite extrémité distale (20D) dudit faisceau (20) puisse éclairer une région de ladite extrémité distale (30D) dudit tube endotrachéal (30), et recueillir une image de ladite région éclairée, et
- un module (10) comprenant un écran (11) et une source de lumière (12), ledit module (10) étant conçu pour être raccordé à l'extrémité proximale (20P) dudit faisceau (20) de fibres optiques pour fournir de la lumière à partir de ladite source de lumière (12) à l'extrémité distale (20D) dudit faisceau (20) de fibres optiques via ladite première fibre optique (21), et pour afficher sur l'écran (11) ladite image recueillie de l'extrémité distale (20D) dudit faisceau (20) de fibres optiques via ladite deuxième fibre optique (22),
**caractérisé en ce que** :
le diamètre externe dudit faisceau (20) de fibres optiques est au moins deux fois plus petit que le diamètre du lumen des voies respiratoires (32) dudit tube endotrachéal (30) de sorte que la ventilation du patient puise s'effectuer tandis que ledit faisceau (20) de fibres optiques est inséré dans ledit tube endotrachéal (30).

2. Système selon la revendication 1, dans lequel ladite extrémité proximale (30P) dudit tube endotrachéal (30) comprend en outre un joint (34) ouvrable et refermable, ledit faisceau (20) de fibres optiques étant fixé audit tube endotrachéal (30) via ledit joint (34) lorsqu'il est inséré dans ledit tube endotrachéal (30).

3. Système selon l'une quelconque des revendications précédentes, dans lequel ledit module (10) comprend en outre des batteries (13) pour fournir de l'énergie à l'écran (11) et à la source de lumière (12).

4. Système selon l'une quelconque des revendications précédentes, comprenant en outre un laryngoscope (50), conçu pour être introduit dans la bouche et les voies respiratoires supérieures dudit patient pour aider à passer ledit tube endotrachéal (30) dans la trachée dudit patient.

5. Système selon l'une quelconque des revendications précédentes, dans lequel le diamètre externe dudit faisceau (20) de fibres optiques est de 4 mm.

6. Système selon l'une quelconque des revendications précédentes, dans lequel ledit tube endotrachéal (30) est un tube endotrachéal de ventilation unipulmonaire.

7. Système selon la revendication 6, dans lequel ledit tube endotrachéal (30) comprend en outre un crochet de carina (35).
